# NEW EUROPEAN PATENT SPECIFICATION

(11) **EP 0 306 206 B2**
(45) Date of publication and mention of the opposition decision: **15.02.1995**
(45) Mention of the grant of the patent: 16.06.1993
(21) Application number: 88307819.8
(22) Date of filing: 24.08.1988
(51) Int. Cl.: G01N 33/53, B01L 3/00, C12M 1/12, G01N 33/543

(54) **Assay device and method**
Testvorrichtung und Verfahren
Dispositif d'essai et procédé

(30) Priority: 27.08.1987 GB 8720253
(43) Date of publication of application: 08.03.1989
(73) Proprietor: COGENT DIAGNOSTICS LIMITED, Edinburgh EH9 3JF (GB)
(72) Inventor: Reid, James Owen, Deans Livingston (GB); Weston, James, Haddington East Lothian (GB)
(74) Representative: Armitage, Ian Michael

(56) References cited:
- EP-A- 0 206 561
- EP-A- 0 269 876
- GB-A- 2 139 519
- GB-A- 2 180 645
- US-A- 4 317 726

## Description

The present invention relates to apparatus for use in immobilising a sample analyte onto a support material so that it can be assayed.

The present invention seeks to provide improvements in diagnostic assays for the detection of low concentrations of analytes from large sample volumes, whereby means are provided for concentrating the analyte onto a defined area of the assay support material and for providing a negative control to assist in detecting non-specific binding and thereby false positive results.

It is well known to detect low concentrations of contaminating microorganisms in for example water, by using conventional techniques to concentrate large sample volume, and then taking aliquots of the concentrate for culture according to standard microbiological techniques. Growth and identification of microorganisms in this way is time consuming, difficult, and frequently takes two to three weeks before results are available.

EP 206561 A describes an assay device in which the sample is funnelled to a small central area of a test filter, but is caused to diffuse laterally through the filter by the effect of a wick in contact with a surrounding area spaced from the central area, so that the sample spreads over a test area which can be viewed in isolation through an aperture in the device. The object is to remove the liquid in the sample laterally through the filter and into the wick, rather than straight through the test area of the filter. The signal generating materials and other reagents are likewise applied to the test area in the same way.

GB 2139519A shows a funnel leading to the central area of a filter disc, the central area of the filter being impermeable so that the liquid only passes into the filter when this central area is moved slightly from contact with the end of the funnel. In effect, therefore, this is a valved filter device, and there is no signal generation associated with the filter disc.

GB 2180645 A shows an assay device with a filter sandwiched between supports, one of which provides openings for admitting sample and reagents. The filter has finger portions extending under these openings, some of which can provide controls. The tests are carried out only on the filter material of the fingers visible through the openings. The adjacent filter material is screened by the supports, but also the liquid sample is diffused laterally from the openings into the main body of the filter material, which acts as a wick.

None of these devices provide the ability to concentrate a substantial amount of sample into a small precisely defined area, and give an easily compared negative control, and possibly also a positive control, in a simple, effective and reliable fashion.

EP 269876A, citable under Ant 54(3)EPC, discloses an assay device in which a removable focuser is in register with a pre-defined reactive area on a test matrix, optionally with passages also communicating with positive and negative control areas on the matrix.

According to one aspect of the present invention there is provided an assay procedure for an analyte which comprises passing a liquid sample suspected of containing the analyte through a filter membrane so that the analyte is retained on the filter, and applying a treatment to the filter to produce a signal on the surface of the filter dependant on the presence of analyte, characterised in that the sample is passed through a defined test area of the filter membrane and the signal producing treatment is applied simultaneously to the test area and an adjacent area of the membrane.

The assay method may comprise detecting the immobilised analyte with a specific antibody. The antibody may be labelled and the label may be an enzyme. The specific antibody may be a monoclonal antibody, and where the label is an enzyme, specific binding of the antibody to the analyte may be detected by means of a suitable substrate, for example 5-bromo-4-chloro-3-indolylphosphate (BCIP) which produces a blue colour when treated with a phosphatase enzyme.

According to another aspect of the present invention there is provided an assay device for an assay of the kind in which an analyte is retained on a filter which is then treated to produce a signal on the surface of the filter dependant on the presence of analyte, the device comprising a filter membrane for retaining analyte from a liquid sample passed through it, and a funnel for delivery of the sample to the membrane, the funnel having an outlet whose cross-sectional area is less than that of the membrane and the funnel outlet being in removable sealing contact with one surface of the membrane whereby the retained analyte is concentrated in a limited test area of the membrane essentially defined by the area of sealing contact with the funnel outlet, the adjacent area of the membrane thereby providing a negative control if the signal producing treatment is simultaneously applied to the whole of the filter membrane. There may be a support (which may be perforated) in contact with the other surface of the filter.

Suitably, the device takes the form of a tube through which the sample and other assay materials can be passed, the filter and its support being arranged transversely across the interior of the tube, the funnel being located, preferably removably, within the tube with its outlet in sealing contact with the surface of the filter remote from the filter support. Preferably the support is detachable from the tube, together with filter, so that the filter can be exposed for inspection after treatment. Suitably, the support has a peripheral weakening allowing it to be broken away from the tube. The end of the tube proximal to the filter support may be placed over a punch-out device which provides means for detaching the support and filter from the tube by rupturing the peripheral weakening. A prefilter component may be provided which is connected or connectable to the inlet of the funnel.

A cap may be provided for locating over the other end of the tube to receive the detached filter and support thereby providing means for their transfer to a holder in which the filter membrane may be displayed. The cap may also be used for stopping the base for reagent addition. Suitably the holder is a card with one or more indentations for holding a disc of assay support material, and a transparent film covering for placing over the disc.

Assay results may be quantified by comparison to a reference chart or to results achieved according to this procedure using test samples of known amounts of the analyte.

A device embodying the invention may comprise a stand having a plurality of mounting members for locating respective tubes during the concentration, the mounting member being connected to a common pump. The stand may also incorporate a punch-out device for the support. The assay can be carried out by blocking off the base of the member and applying reagents to produce a signal.

In order that the present invention may be more clearly understood one embodiment will be further illustrated by way of example only and with reference to the drawings wherein:

Fig. 1 is a diagrammatic cross-sectional representation of the assay device.

Fig. 2 is a diagrammatic cross-sectional representation of the basal region of the assay device with a punch-out device for removal of the filter membrane.

Fig. 3 is a diagram showing successive stages in the removal of the filter membrane from the assay device and location of the filter membrane in a holder.

Fig. 4 is a diagrammatic representation in plan view of a filter showing a positive assay result with a non-specific background.

Fig. 5 is a perspective view of an apparatus embodying the assay device of the present invention.

An assay and assay device of this type may be used for the detection of microbial contaminants such as Legionella species in water samples etc. Such contaminants, although present in very low concentrations are potentially extremely pathogenic. Therefore, there is a need for an assay system which concentrates these microbes, thereby rendering them readily detectable. Furthermore, it would be desirable for such an assay system to have some kind of negative and positive control, to assist in distinguishing true positive results from false positive results e.g. occurring as a result of non-specific binding and false negative results as a result of e.g. assay or reagent malfunction.

In a typical assay procedure, a large sample volume of water is first subjected to conventional coarse filtering and concentration techniques so as to produce a small sample of, for example, 20mls containing the concentrated analyte of interest, e.g. Legionella species, which is relatively free from debris material and which provides aliquots for assaying.

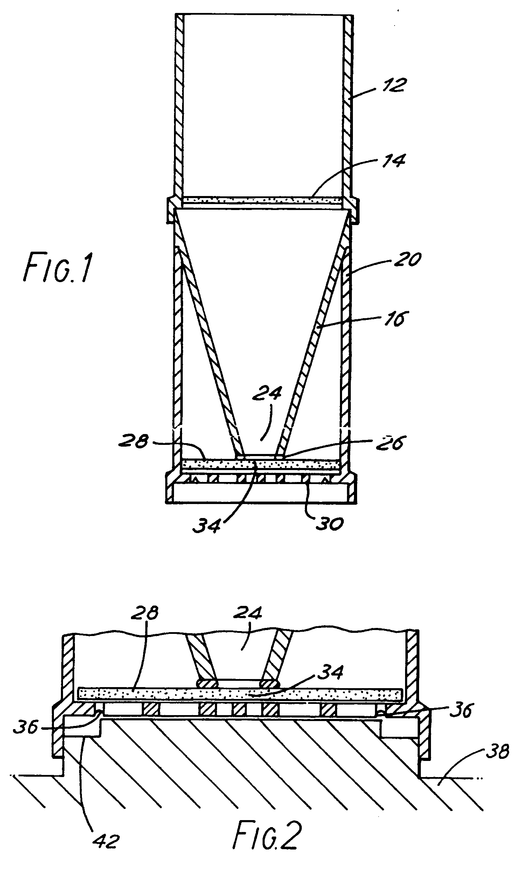

Referring to Figs 1 and 2, an assay device comprises a prefilter component in the form of a tube 12 having at its base a coarse filter 14. This prefilter component is a tight push fit-in connected to the inlet of a funnel 16. The funnel 16 is removably located within a tube 20. The funnel has a narrow outlet 24, terminating in a rubber gasket ring 26 by which it makes sealing contact with an area 34 in the centre of a filter membrane 28 which extends across the tube 20. The filter membrane 28 rests on a perforated support 30 which is integral with the tube 20. The filter membrane 28 is compressed between the gasket ring 26 and the support 30 to ensure a peripheral seal around the outlet 24 and prevent lateral diffusion of the sample in the membrane. The support 30 has peripheral weakening 36 so that it can be broken away from the tube. The base of the tube 20 can be connected to a suitable pump for drawing liquid sample through the device, or capped for assaying.

In use, the device is placed in position on a pump and an aliquot of the preconcentrated sample is dispensed into the prefilter 12. This sample then passes through the coarse filter, 14 which acts to remove any further debris, and thence into the funnel 16 and through the small central area 34 of the filter membrane 28. The filter membrane 28 is able to immobilise analytes such as Legionella, whilst liquid e.g. water passes through it to the pump outlet. In this way, all analyte molecules originally contained in the initial large sample volume are concentrated onto the small area 34 of the filter membrane. The pump is now switched off and the prefilter component 12, together with the funnel 16 are then removed from the tube 20, leaving the tube 20 with the filter membrane 28 at its base.

The remaining assay process is conducted within the tube 20 and comprises the following steps applied to the whole of the filter membrane 28:
a) adding an enzyme-labelled monoclonal antibody wherein the antibody is specific for the analyte of interest;
b) washing off any unbound enzyme-labelled monoclonal antibody; and
c) adding a substrate suitable for the enzyme, whereby interaction with the enzyme causes an insoluble coloured precipitate to be deposited on the filter membrane 28 where the enzyme label has been bound by specific interactions between the analyte and monoclonal antibody. A suitable enzyme is an alkaline phosphatase, for which a suitable substrate is 5-bromo-4-chloro-3-indolylphosphate, which gives a blue precipitate.

The end of the tube 20 adjacent the filter membrane 28 is then placed on a punch-out device 38 which has a step like configuration 42 at its outer margin, so that when the tube 20 is pushed down onto the punch-out device 38, the top step ruptures the support 30 at its peripheral weakening 36 and also detaches the filter membrane 28. Because the filter is removed by rupturing its attachment to the tube, the filter cannot be replaced, and thus there is no possibility of inadvertent re-use.

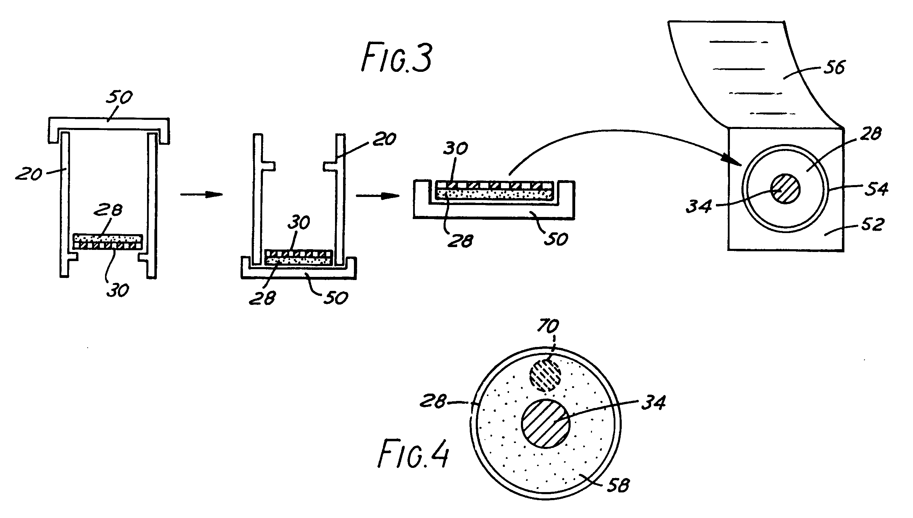

As shown in Figure 3, the detached filter membrane and its support 30 can be collected in a cap 50 placed over the upper end of the tube 20 by inverting the tube. The cap 50 is then inverted over a card 52 having a suitable indentation 54 for receiving the filter 28 and support 30. The filter will of course be uppermost and therefore exposed to view. A flap 56 of transparent film is placed over the card to retain the filter while permitting its inspection. In this way a permanent record of the assay result may be kept.

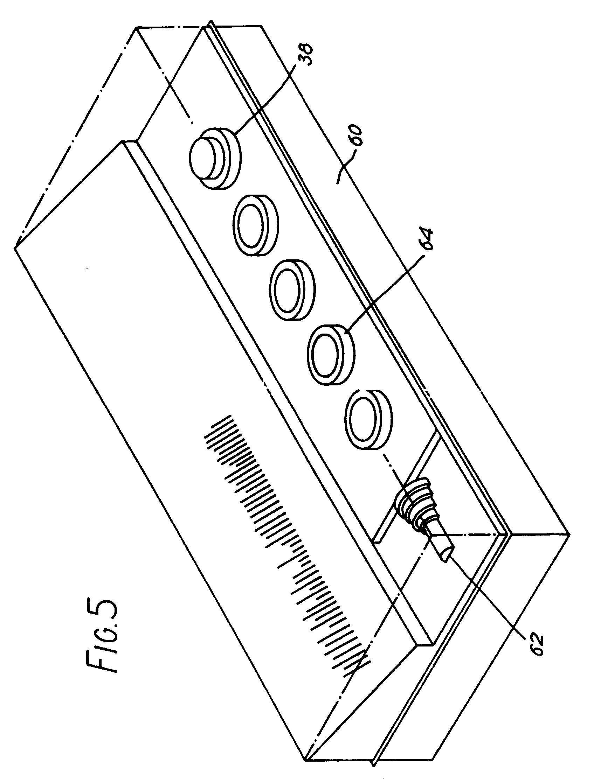

Figure 4 shows a typical positive assay result whereby colour produced by the deposition of an insoluble coloured precipitate in response to the action of antibody-bound enzyme with substrate, is generated in the test area 34 and is surrounded by a negative control area 58. By comparison with a range of standard assay discs, e.g. produced by assaying known amounts of the analyte according to this procedure, a reasonably accurate quantitative result indicating the original concentration of analyte in the test sample may be obtained. Alternatively the colour can be compared to a pre-calibrated colour comparator.

The present invention has the advantage that by using the funnel the analyte is further concentrated in a smaller area of the filter, thereby achieving a correspondingly higher degree of sensitivity. For example if the diameter of the funnel outlet is slightly less than one-third the diameter of the filter, there will be a ten-fold increase in concentration of analyte compound with the use of the whole filter. Moreover, a further advantage is obtained in that the area 58 of the filter surrounding the test area 34 is subjected simultaneously, and therefore under the same conditions, to the assay treatment and provides a negative control against which the signal in the test area can be compared. Thus, if a similar degree of signal is observed in both areas, this is likely to be a negative result; whereas without the control, the signal in the test area might be regarded as a positive result. This in particular enables the entire filter membrane to be pre-coated with a specific binding member, e.g. antibody, for the analyte. Any non-specific binding to this material by the reagents in the subsequent treatment will be distinguished by the contrast between the signal in the test area and that in the surrounding area.

In addition, however, a positive control may be obtained by providing on the filter membrane 28, in advance of the assay, a spot or other limited area 70 (see Fig. 4) containing a sample of the analyte of interest or another method of producing the same response. This spot will be in the area 58 surrounding the test area 34. Thus, the development of a signal in this positive control spot 70 will confirm that the assay procedure is effective, and thereby give greater confidence to a negative result in the test area 34. Indeed, the provision of a number of positive control spots or areas, carrying different concentrations of analyte, could provide a self-contained calibration for the signal produced in the test area, in addition to or instead of a chart of standard assay discs as referred to above. A significant feature of the present invention is that the sample is confined to a discrete area of the filter, and the whole of the filter is then treated with reagent, thereby giving a true negative control. This is distinct from arrangements in which a filter is pretreated in discrete areas with a specific binding member for the analyte, and the entire filter subject to the sample.

The test area need not be circular, nor need it be in the centre of the filter. For some purposes it may be desirable to have the outlet of the funnel in the form of a slot, to provide a bar, rather than a spot, of test material.

Figure 5 shows a suitable apparatus using the device of the present invention, and comprises a stand 60 having an outlet means 62 for connecting to a pump. A plurality of mounting members 64 provide means for securing one or more of the described assay devices via their tubes 20. Each mounting member 64 connects to the pump outlet 62, and when any are not required they can be blanked off, e.g. with a removable cap. A punch-out device 38 is also incorporated, to provide means for removal of the discs of filter membrane 28 from the tubes 20.

If desired, the assay tube 12 can be provided with graduations, e.g. at 10 ml, 20 ml, 30 ml levels, to facilitate the addition of known volumes of reagents. The prefilter tube 12 may likewise be graduated or be of known volume, to facilitate the addition of a known amount of sample.

The assay tube 12 may be provided with a small aperture in its side wall near the top (remote from the membrane) to facilitate venting and possible priming with reagent. In particular, after suction is applied to the tube, the vent aperture can be opened to release any partial vacuum in the space between the tube and the funnel. Conveniently the aperture can be normally closed by the funnel or the prefilter unit, and opened by rotation of the funnel or prefilter unit to bring an aperture therein in register with the aperture in the tube.

The non-replaceability of the filter can be provided by means of the frangible support as illustrated, or it could be provided by other means, such as, a self-destruct twisting action to remove the filter. Such an action would, for example, have the effect of permanently destroying a seal or a screw-thread, and thereby preventing effective refitting of the filter in the tube.

## Claims

1. An assay device of the kind in which an analyte is retained on a filter which is then treated to produce a signal on the surface of the filter dependant on the presence of analyte, characterised in that the device comprises a tube (20) through which a liquid sample and other assay materials can be passed, a filter membrane (28) for retaining analyte from a liquid sample passed through it, a perforate support (30) therefor being arranged transversely across the interior of the tube, and a funnel (16) for delivery of the sample to the membrane, the funnel being removably located within the tube and having an outlet (24) whose cross-sectional area is less than that of the membrane and the funnel outlet being in removable sealing contact with one surface of the membrane, whereby the retained analyte is concentrated in a limited test area (34) of the membrane essentially defined by the area of sealing contact with the funnel outlet, the adjacent area (58) of the membrane thereby providing a negative control if the signal producing treatment is simultaneously applied to the whole of the filter membrane, the membrane and support being attached to the tube by means which are rupturable to allow the support with the membrane to be non-replaceably detached so that the membrane can be exposed for inspection after treatment.

2. As assay device according to claim 1 which includes a cap (50) for locating over one end of the tube (20) to receive the detached filter membrane (28).

3. An assay device according to claim 2 wherein the cap is also designed to be capable of plugging the other end of the tube to facilitate reagent retention.

4. An assay device according to any one of claims 1 to 3 in combination with a holder (52) in which the filter membrane may be displayed.

5. An assay device according to claim 4 wherein the holder is in the form of a card (52) with one or more indentations (54) to receive the filter membrane (28) and a transparent film covering (56) for placing over the membrane.

6. An assay device according to any one of the preceding claims which includes one or more prefilter components (12, 14) connected or connectable to the inlet of the funnel.

7. An assay device according to any one of the preceding claims in which the entire filter membrane is coated or impregnated with a specific binding member for the analyte.

8. An assay procedure for an analyte of the kind which comprises passing a liquid sample suspected of containing the analyte through a tube containing a transversely extending filter membrane mounted on a perforate support so that the analyte is retained on the membrane, and applying a treatment to the filter to produce a signal on the surface of the filter dependant on the presence of analyte; characterised in that the procedure comprises:
(i) passing the sample through a defined test area of the filter membrane, and
(ii) applying the signal producing treatment simultaneously to the test area and an adjacent area of the filter so as to provide for the signal a negative control in said adjacent area of the filter, the support carrying the filter membrane then being non-replaceably detached from the tube and removed for inspection of the membrane.

9. An assay procedure according to claim 8 wherein the filter has a positive control area within the area of the filter subjected to the signal producing treatment, but separate from the test area, and therefore not contacted by the sample, the positive control area having been provided in advance with means for generating a positive response when subject to said signal producing treatment.

10. An assay procedure according to claim 8 or claim 9 wherein the entire area of the filter has been pre-coated or impregnated with a specific binding member for the analyte.

## Patentansprüche

1. Prüf- bzw. Untersuchungs- bzw. Bestimmungsvorrichtung der Art, bei der ein Analyt auf einem Filter zurückgehalten wird, der dann behandelt wird, um in Abhängigkeit von der Gegenwart von Analyt ein Signal auf der Oberfläche des Filters zu erzeugen, dadurch gekennzeichnet, daß die Vorrichtung ein Rohr (20), durch das eine flüssige Probe und andere Prüf- bzw. Untersuchungs- bzw. Bestimmungsmaterialien hindurchgeschickt werden können, eine Filtermembran (28) zum Zurückhalten von Analyt von einer flüssigen Probe, die durch sie hindurchgegangen ist, einen perforierten Träger (30) dafür, der quer über das Innere des Rohres angeordnet ist, und einen Trichter (16) zum Abgeben der Probe an die Membran umfaßt, wobei der Trichter abnehmbar innerhalb des Rohres angeordnet ist und einen Auslaß (24) aufweist, dessen Querschnittsfläche kleiner als jene der Membran ist, und der Trichterauslaß sich in entfernbarem Dichtungskontakt mit einer Oberfläche der Membran befindet, wodurch der zurückgehaltene Analyt in einem begrenzten Prüfbereich (34) der Membran eingeengt wird, der im wesentlichen durch den Bereich des Dichtungskontakts mit dem Trichterauslaß begrenzt bzw. bestimmt ist, wodurch der angrenzende Bereich (58) der Membran eine negative Kontrolle bietet, wenn die signalerzeugende Behandlung gleichzeitig auf die gesamte Filtermembran angewandt wird, wobei Membran und Träger am Rohr durch Mittel befestigt sind, die gebrochen werden können, um zu ermöglichen, daß der Träger mit der Membran nicht wieder einsetzbar losgelöst bzw. abgenommen wird, sodaß die Membran nach der Behandlung zwecks Untersuchung freigelegt werden kann.

2. Prüf- bzw. Untersuchungs- bzw. Bestimmungsvorrichtung nach Anspruch 1, die eine Kappe bzw. einen Deckel (50) zum Anordnen über einem Ende des Rohres (20) einschließt, um die losgelöste bzw. abgenommene Filtermembran (28) aufzunehmen.

3. Prüf- bzw. Untersuchungs- bzw. Bestimmungsvorrichtung nach Anspruch 2, worin die Kappe bzw. der Deckel auch dazu bestimmt ist, das andere Ende des Rohres zustöpseln zu können, um das Zurückhalten von Reagens zu erleichtern.

4. Prüf- bzw. Untersuchungs- bzw. Bestimmungsvorrichtung nach einem der Ansprüche 1 bis 3 in Kombination mit einer Haltevorrichtung (52), in der die Filtermembran ausgelegt werden kann.

5. Prüf- bzw. Untersuchungs- bzw. Bestimmungsvorrichtung nach Anspruch 4, worin die Haltevorrichtung die Form einer Karte (52) mit einer oder mehreren Einkerbungen bzw. Vertiefungen (54) zum Aufnehmen der Filtermembran (28) und einer transparenten Filmabdeckung (56) zum Legen über die Membran hat.

6. Prüf- bzw. Untersuchungs- bzw. Bestimmungsvorrichtung nach einem der vorhergehenen Ansprüche, welche einen oder mehrere Vorfilterbestandteile (12,14) einschließt, die mit dem Einlaß des Trichters verbunden oder verbindbar sind.

7. Prüf- bzw. Untersuchungs- bzw. Bestimmungsvorrichtung nach einem der vorhergehenen Ansprüche, in der die gesamte Filtermembran mit einem speziellen Bindungselement für den Analyten beschichtet oder imprägniert ist.

8. Prüf- bzw. Untersuchungs- bzw. Bestimmungsverfahren für einen Analyten von der Art, die das Hindurchschicken einer flüssigen Probe, von der angenommen wird, daß sie den Analyten enthält, durch ein Rohr, das eine sich quer erstreckende Filtermembran enthält, die auf einem perforierten Träger montiert ist, so daß der Analyt auf der Membran zurückgehalten wird, sowie das Anwenden einer Behandlung auf den Filter umfaßt, um ein von der Gegenwart von Analyt abhängiges Signal auf der Oberfläche des Filters zu erzeugen; dadurch gekennzeichnet, daß das Verfahren umfaßt:
(i) das Hindurchschicken der Probe durch einen begrenzten Prüf- bzw. Testbereich der Filtermembran, und
(ii) das Anwenden der signalerzeugenden Behandlung gleichzeitig auf den Prüf- bzw. Testbereich und einen angrenzenden Bereich des Filters, um im genannten angrenzenden Bereich des Filters eine negative Kontrolle für das Signal zu schaffen, wobei der die Filtermembran tragende Träger dann nicht wieder einsetzbar vom Rohr losgelöst bzw. abgenommen und zur Untersuchung der Membran entfernt wird.

9. Prüf- bzw. Untersuchungs- bzw. Bestimmungsverfahren nach Anspruch 8, worin der Filter einen positiven Kontrollbereich aufweist, der sich innerhalb des Bereichs des Filters, der der signalerzeugenden Behandlung unterworfen wird, aber getrennt vom Prüf- bzw. Testbereich befindet und daher nicht von der Probe berührt wird, wobei der positive Kontrollbereich im vorhinein mit Mitteln zum Erzeugen einer positiven Reaktion, wenn er der genannten signalerzeugenden Behandlung unterworfen wird, versehen worden ist.

10. Prüf- bzw. Untersuchungs- bzw. Bestimmungsverfahren nach Anspruch 8 oder 9, worin der gesamte Bereich des Filters mit einem speziellen Bindungselement für den Analyten vorbeschichtet oder imprägniert worden ist.

## Revendications

1. Dispositif d'essai du type où un analyte est retenu sur un filtre qui est alors traité pour produire un signal à la surface du filtre dépendant de la présence de l'analyte, caractérisé en ce que le dispositif comprend un tube (20) par lequel peuvent passer un échantillon liquide et d'autres matériels d'essai, une membrane de filtre (28) pour retenir l'analyte d'un échantillon liquide l'ayant traversé, son support perforé (30) étant agencé transversalement à l'intérieur du tube, et un entonnoir (16) pour fourniture de l'échantillon à la membrane, l'entonnoir étant amovible dans le tube et ayant une sortie (24) dont l'aire en section transversale est plus petite que celle de la membrane et la sortie de l'entonnoir étant en contact d'obturation amovible avec une surface de la membrane, ainsi l'analyte retenu est concentré dans une aire limitée de test (34) sur la membrane qui est essentiellement définie par l'aire de contact d'obturation avec la sortie de l'entonnoir, l'aire adjacente (58) de la membrane formant ainsi un contrôle négatif si le traitement de production du signal est simultanément appliqué à la totalité de la membrane du filtre, la membrane et le support étant attachés au tube par des moyens qui peuvent être rompus pour permettre au support avec la membrane d'être détaché sans pouvoir être remis en place de façon que la membrane puisse être exposée pour inspection après traitement.

2. Dispositif d'essai selon la revendication 1 qui comprend un capuchon (50) à placer sur une extrémité du tube (20) pour recevoir la membrane de filtre (28) détachée.

3. Dispositif d'essai selon la revendication 2 où le capuchon est également conçu pour pouvoir boucher l'autre extrémité du tube afin de faciliter la retenue des réactifs.

4. Dispositif d'essai selon l'une des revendications 1 à 3 en combinaison avec un soutien (52) dans lequel peut être présentée la membrane du filtre.

5. Dispositif d'essai selon la revendication 4 où le soutien a la forme d'une carte (52) avec un ou plusieurs creux (54) pour recevoir la membrane de filtre (28) et un recouvrement en film transparent (56) à placer sur la membrane.

6. Dispositif d'essai selon l'une quelconque des revendications précédentes, qui comporte un ou plusieurs composants de préfiltre (12, 14) qui sont connectés ou qui peuvent être connectés à l'entrée de l'entonnoir.

7. Dispositif d'essai selon l'une quelconque des revendications précédentes où la totalité de la membrane du filtre est enduite ou imprégnée d'un organe spécifique de liaison de l'analyte.

8. Processus d'essai pour un analyte, du type qui consiste à faire passer un échantillon liquide suspecté de contenir l'analyte à travers un tube comprenant une membrane de filtre s'étendant transversalement, montée sur un support perforé, de manière que l'analyte soit retenu sur la membrane et à appliquer un traitement au filtre pour produire un signal à la surface du filtre dépendant de la présence de l'analyte, caractérisé en ce que le processus comprend :
(i) le passage de l'échantillon à travers une aire définie de test de la membrane de filtre, et
(ii) l'application d'un traitement de production d'un signal simultanément à l'aire de test et à une aire adjacente du filtre afin de produire, pour le signal, un contrôle négatif dans ladite aire adjacente du filtre, le support portant la membrane de filtre étant alors détaché du tube sans pouvoir être remis en place et retiré pour l'inspection de la membrane.

9. Processus d'essai selon la revendication 8 où le filtre a une aire de contrôle positif dans l'aire du filtre qui est sujette au traitement de production du signal, mais qui est séparée de l'aire de test et par conséquent qui n'est pas contactée par l'échantillon, l'aire de contrôle positif ayant été pourvue à l'avance d'un moyen pour produire une réponse positive lorsqu'elle est soumise au traitement de production du signal.

10. Processus d'essai selon la revendication 8 ou la revendication 9 où toute aire du filtre a été pré-enduite ou imprégnée d'un organe spécifique de liaison de l'analyte.
